# EUROPEAN PATENT APPLICATION

(11) **EP 4 834 243 A1**
(43) Date of publication of application: **04.11.2026**
(21) Application number: 24910962.0
(22) Date of filing: 20.12.2024
(51) Int. Cl.: A61B 17/04

(54) **SUTURE KNOT TYING SYSTEM**

(30) Priority: 28.12.2023 CN 202311847758
(71) Applicant: Shenzhen Bihe Medical Co., Ltd., Shenzhen, Guangdong 518063 (CN)
(72) Inventor: TANG, Lin, Shenzhen, Guangdong 518063 (CN); LI, Siyi, Shenzhen, Guangdong 518063 (CN); TANG, Huiqiang, Shenzhen, Guangdong 518063 (CN); SHAO, Ge, Shenzhen, Guangdong 518063 (CN)
(74) Representative: Michalski Hüttermann & Partner mbB
(86) International application number: PCT/CN2024/140972
(87) International publication number: WO 2025/140033

(57) **Abstract**

Disclosed in the present invention is a suture knot tying system, which comprises a driving mechanism, a sheath, a screw head assembly provided at the distal end of the sheath, a blade cannula provided inside the sheath and a cutting portion provided inside the sheath. The screw head assembly comprises a suture channel communicated with the sheath; the distal end of the blade cannula is provided with a blade portion in contact fit with the cutting portion; and a suture can pass through the suture channel and extend into the sheath and is located between the cutting portion and the blade portion. The proximal end of the blade cannula is connected to the driving mechanism. During the process of operating the driving mechanism, the driving mechanism can simultaneously drive the blade cannula to move in the axial direction of the sheath relative to the sheath and drive the blade cannula to rotate around its own axis. The suture knot tying system disclosed by the present invention requires a low pressure for cutting sutures, so as to reduce a pushing force that the blade cannula applies on the screw head assembly and a pressure between the blade portion and the cutting portion, thereby reducing the probability of damage to the blade cannula or the sheath.

## Description

### Technical Field

The present invention relates to the technical field of medical instruments, and in particular, to a suture knot locking system.

### Background Art

Generally, sutures need to be knotted and secured in surgery. Since surgery is typically carried out under the eyes of a surgeon, the sutures are usually knotted manually by the surgeon. However, minimally invasive surgery and interventional operation are increasingly widely applied thanks to their advantages of minimal trauma and rapid recovery. Transcatheter interventional surgery is taken as an example. An instrument such as a catheter needs to be inserted into the body of a patient to a predetermined position for suturing. After the suturing operation is completed, a knotting instrument is used to grasp a suture and insert the suture to a suturing site. The knotting instrument is used to be remotely operated outside the body of the patient to complete knotting of the suture inside the body of the patient, cut off the knotted suture, and withdraw a suture, which is left after cutting, along with the knotting instrument from the body of the patient, thus completing the surgery.

For the cutting of the suture, existing equipment is usually provided with a blade that is manually pushed to move axially. A distal end of the blade presses a plane of a proximal side of a chuck to cut off the suture. However, it is significantly difficult to cut off the suture by pressing because it is hard to control a desired pressure. An insufficient pressure cannot cut off the suture. An excessive pressure may damage a cutting edge and generate fragments, or may stretch a sheath to deform. This endangers the health of the patient.

### Summary of the Invention

A technical problem to be solved in the present invention is a problem that it is significantly difficult to cut off a suture only by a pressure between a blade and a chuck and there is a potential safety hazard. For the defects in the existing art, a suture knot locking system is provided.

Solving the technical problem of the present invention is implemented through the following technical solutions:
A suture knot locking system includes a driving mechanism, a sheath, a tying head assembly disposed at a distal end of the sheath, a blade tube disposed inside the sheath, and a cutting portion disposed inside the sheath. The tying head assembly includes a suture threading channel communicated with the sheath. A cutting edge that is in contact fit with the cutting portion is disposed at a distal end of the blade tube. A suture can extend into the sheath through the suture threading channel and be located between the cutting portion and the cutting edge. A proximal end of the blade tube is connected to the driving mechanism. In a process of operating the driving mechanism, the driving mechanism can simultaneously drive the blade tube to move in an axial direction of the sheath relative to the sheath and drive the blade tube to rotate around an axis of the blade tube.

According to the above suture knot locking system, the driving mechanism simultaneously drives the blade tube to move in the axial direction of the sheath relative to the sheath and rotate around the own axis, the suture can be clamped between the cutting edge and the cutting portion of the blade tube, and the cutting edge can cut the suture in a manner of rotating relative to the cutting portion, so that the cutting edge simultaneously applies a pressure and a cutting force to the suture, thus enhancing the cutting effect of the cutting edge on the suture and lowering the difficulty of cutting off the suture. Moreover, compared with a method for cutting off the suture by pressing, the method for cutting the suture by rotating the cutting edge relative to the cutting portion requires low pressure to cut the suture. This can reduce the pushing force applied by the blade tube to the tying head assembly and the pressure between the cutting edge and the cutting portion, and reduce the probability of damage to the blade tube or sheath.

### Brief Description of the Drawings

The present invention is further described below with reference to the accompanying drawings and embodiments. In the accompanying drawings:
FIG. 1 is a schematic structural diagram of an exemplary suture knot locking system according to the present invention;
FIG. 2 is a schematic exploded view of parts of an exemplary suture knot locking system according to the present invention;
FIG. 3 is a partial cross-sectional view of an exemplary suture knot locking system according to the present invention;
FIG. 4 is a partial cross-sectional view of a tying head assembly, a sheath, a support sleeve, a blade tube, and a push rod of an exemplary suture knot locking system according to the present invention;
FIG. 5 is a cross-sectional view of a tying head seat and a tying head of an exemplary suture knot locking system according to the present invention;
FIG. 6 is a schematic structural diagram of the assembling of a driving mechanism of an exemplary suture knot locking system according to the present invention;
FIG. 7 is a schematic exploded view of parts of a driving mechanism of an exemplary suture knot locking system according to the present invention;
FIG. 8 is a schematic structural diagram of an exemplary suture knot locking system according to the present invention;
FIG. 9 is a partial enlarged schematic view of portion A in FIG. 8;
FIG. 10 is a schematic structural diagram of a support sleeve and a tube clamp in an exemplary suture knot locking system according to the present invention;
FIG. 11 is a schematic structural diagram of a blade tube in an exemplary suture knot locking system according to the present invention;
FIG. 12 is a partial cross-sectional view of a tying head assembly, a sheath, a support sleeve, a blade tube, and a push rod of an exemplary suture knot locking system according to the present invention;
FIG. 13 is a cross-sectional view of a tying head seat and a tying head of an exemplary suture knot locking system according to the present invention; and
FIG. 14 is a schematic diagram of a tying head, a tying pin, and a suture of an exemplary suture knot locking system according to the present invention, after completing knot locking and cutting on the suture.

In the accompanying drawings, representations of numerals are as follows:
100: suture knot locking system;
10: handle; 11: upper shell; 12: lower shell; 13: end cover; 14: stress relief sleeve; 141: fixing portion; 142: tubular portion; 101: accommodating cavity; 102: first sliding structure; 103: first fixing slot; 104: end cover mounting portion; 105: second fixing slot; 106: support portion; 1061: insertion slot;
20: sheath; 21: second suture passing hole;
30: tying head assembly; 31: tying head seat; 311: first stepped section; 312: second stepped section; 313: third stepped section; 314: step surface; 315: seat hole; 3151: first stepped segment; 3152: second stepped segment; 3153: clamping slot; 3154: ringlike slot; 316: cutting portion; 317: stop slot; 32: tying head; 321: pin hole; 322: ringlike protrusion; 323: stop portion; 33: tying pin; 331: main body portion; 332: limiting portion; 333: raised portion; 301: suture threading channel; 34: knot locking member;
40: blade tube; 41: main body section; 42: cutting section; 421: cutting edge; 401: first snap-in slot;
50: push rod;
60: driving mechanism; 61; screw-in member; 611: slidable connection portion; 6111: sliding chute; 6112: first pin shaft hole; 612: threaded fit portion; 613: connection rod portion; 6131: rotatable limiting portion; 6132: limiting gap; 614: through hole; 62: fixing member; 621: threaded hole; 63: moving member; 631: rotatable connection portion; 6311: rotatable limiting hole; 6312: second pin shaft hole; 632: fixed connection portion; 6321: first fastening hole; 6322: second fastening hole; 633: first side wall; 634: second side wall; 6301: second sliding structure; 6302: avoidance slot; 64: knob; 641: sliding block;
70: support sleeve; 71: first suture passing hole; 72: second snap-in slot;
80: connection sleeve;
90: suture guider; 91: suture guide grip; 92: suture guide wire; 911: suspended arm portion;
1001: first pin shaft; 1002: second pin shaft; 1003: tube clamp; 10031: third snap-in slot; 1004: fastening screw; and
200: suture.

### Detailed Description of the Invention

To provide a clearer understanding of the technical features, objectives, and effects of the present invention, specific embodiments of the present invention are described in detail with reference to the accompanying drawings.

The exemplary embodiments of the present disclosure will be described in more detail below with reference to the accompanying drawings. Although the accompanying drawings show the exemplary embodiments of the present disclosure, it should be understood that the present disclosure can be implemented in various forms, and should not be limited to the embodiments stated herein. Rather, these embodiments are provided for understanding the present disclosure more thoroughly, and can completely transfer the scope of the present disclosure to those skilled in the art.

It should be understood that the terms used herein are only for the purpose of describing specific exemplary embodiments and are not intended to be restrictive. Unless otherwise explicitly stated in the context, the singular forms such as "a/an", "one", and "the" used herein can also indicate a plural form. The terms "include", "contain", "containing", and "have" are inclusive and therefore indicate the existence of the stated features, steps, operations, elements, and/or components, but do not exclude the existence or addition of one or more other features, steps, operations, elements, components, and/or their combinations. The method steps, processes, and operations described herein are not interpreted as requiring them to be executed in a specific order described or explained, unless an order of execution is clearly indicated. It should also be understood that other or alternative steps can be used.

Although the terms first, second, third, etc. may be used herein to describe multiple elements, components, regions, layers, and/or sections, these elements, components, regions, layers, and/or sections should not be limited by these terms. These terms can only be used to distinguish one element, component, region, layer, or section from another element, component, region, layer, or section. Unless explicitly stated in the context, terms such as "first", "second", and the like used herein do not imply an order or a sequence. Therefore, a first element, component, region, layer, or section discussed below may be referred to as a second element, component, region, layer, or section without departing from the teachings of the exemplary embodiments.

For ease of description, spatial relative relationship terms can be used herein to describe a relationship between an element or feature shown in the figure relative to another element or feature, such as "internal", "external", "inner side", "outer side", "beneath", "below", "on", and "above". This spatial relative relationship term means including different orientations of a device in use or operation, in addition to the orientations depicted in the figures. For example, if a device in a drawing is flipped, an element described as being "below other elements or features" or "under other elements or features" will subsequently be oriented as being "above other elements or features" or "over other elements or features". Therefore, the exemplary term "below" can include two orientations: "above" and "below". The device can be oriented in other ways (rotated 90 degrees or in other directions) and space-correlated descriptors used herein can also be explained correspondingly.

It should be noted that "distal end" and "proximal end" are used as localizers. The localizers are conventional terms in the field of interventional medical instruments. A "distal end" is defined as an end that is far away from an operator in a surgical process, and a "proximal end" is defined as an end that is close to the operator in the surgical process. Axial direction is a direction parallel to a connection line between a center of a distal end of a medical instrument and a center of a proximal end of the medical instrument. Radial direction is a direction perpendicular to the aforementioned axial direction.

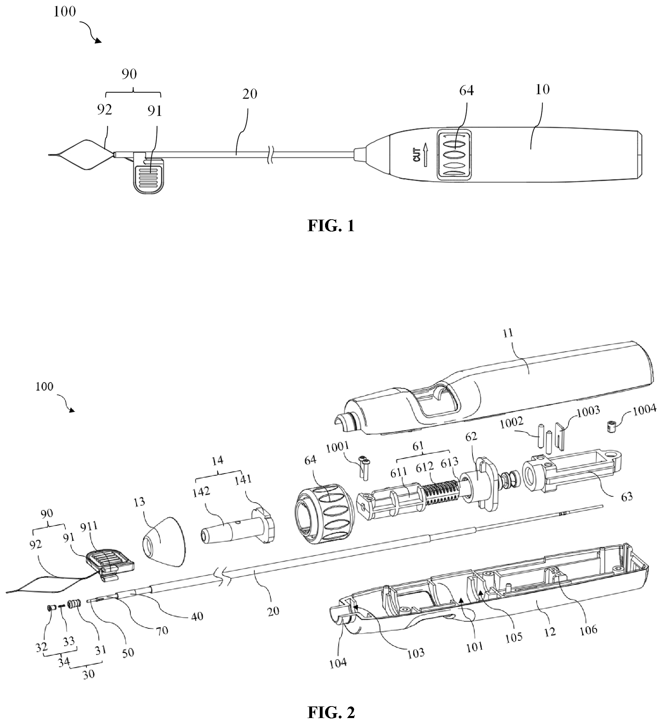

Referring to FIG. 1, FIG. 2, FIG. 4, FIG. 5, and FIG. 6, this embodiment provides a suture knot locking system 100, including a sheath 20, a tying head assembly 30, a handle 10, a blade tube 40, a push rod 50, and a driving mechanism 60. The tying head assembly 30 is disposed on the distal side of the sheath 20. The tying head assembly 30 is provided with a suture threading channel 301 communicated with the sheath 20. The suture 200 can extend into the sheath 20 via the suture threading channel 301. The proximal end of the sheath 20 is connected to the handle 10. The push rod 50 is disposed inside the sheath 20 and can move in an axial direction of the sheath 20 relative to the sheath 20. The driving mechanism 60 is movably disposed on the handle 10. The proximal end of the push rod 50 is connected to the driving mechanism 60. The driving mechanism 60 can drive the push rod 50 to move in the axial direction of the sheath 20 during movement. When the push rod 50 moves toward the distal end in the axial direction of the sheath 20, the distal end of the push rod 50 can press the tying head assembly 30. Under the pressing action of the push rod 50, the tying head assembly 30 can transition from an initial state to a knot locking state, thereby locking the suture threaded in the tying head assembly 30. In other embodiments, the suture knot locking system 100 may not include the handle 10, as long as the driving mechanism 60 can control the blade tube 40 and the push rod 50.

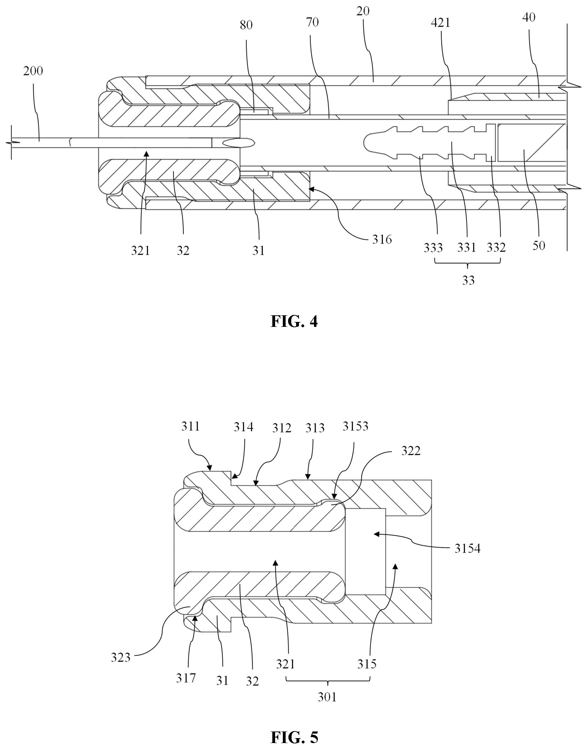

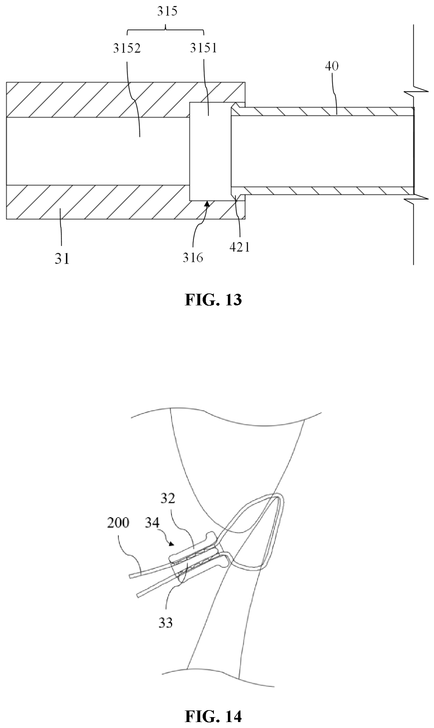

It should be noted that as shown in FIG. 4 and FIG. 5, the initial state of the tying head assembly 30 refers to a state in which the suture threading channel 301 is unobstructed. In this case, the suture 200 can freely enter and exit the suture threading channel 301, or the suture 200 can freely move within the suture threading channel 301. As shown in FIG. 5 and FIG. 14, the knot locking state of the tying head assembly 30 refers to a state in which the suture threading channel 301 is closed and the suture is locked. In this case, the suture 200 is fixedly connected to at least some components in the tying head assembly 30. It can be understood that the tying head assembly 30 can be configured in various structural forms to achieve the transitioning from the initial state to the knot locking state. For example, in some embodiments, the tying head assembly 30 can undergo overall deformation under the pressing action of the distal end of the push rod 50, so that the suture threading channel 301 bends and deforms, and an inner wall of the suture threading channel 301 radially deforms and clamps and fixes the suture 200. Alternatively, in some other implementations, by cooperation of different components in the tying head assembly 30, some components in the tying head assembly 30 are moved and block the suture threading channel 301 under the pressing action of the push rod 50, so as to clamp and fix the suture 200 inside the suture threading channel 301. Alternatively, in another embodiment, the push rod 50 or another element does not need to press the tying head assembly 30 to achieve the knot locking state. The tying head assembly 30 can be always in an automatic knot locking state. For example, there is a channel capable of being automatically locked inside the tying head assembly 30, and the inner diameter of the channel is less than the outer diameter of the suture. The interior of the tying head assembly 30 is made of an elastic material. The suture 200 is threaded into the channel. The suture 200 and the tying head assembly 30 can be fixed to each other without an external force. Then, the tying head assembly 30 is pushed through the push rod 50 to push out at least part of the tying head assembly 30. Alternatively, in another embodiment, the push rod 50 may not be provided, and the tying head assembly 30 can always be in the knot locking state. For example, there is a channel capable of being automatically locked inside the tying head assembly 30, and the inner diameter of the channel is less than the outer diameter of the suture. The interior of the tying head assembly 30 is made of an elastic material. The suture 200 is threaded into the channel. The suture 200 and the tying head assembly 30 can be fixed to each other without an external force. Then, the sheath 20 is withdrawn, and the tying head assembly 30 can fall off from the sheath 20.

Further, as shown in FIG. 4, the tying head assembly 30 further includes a cutting portion 316 located inside the sheath 20. The blade tube 40 surrounds the push rod 50 and is located inside the sheath 20. The blade tube 40 can move relative to the sheath 20 in the axial direction of the sheath 20. A cutting edge 421 is disposed at the distal end of the blade tube 40 and is opposite to the cutting portion 316. The cutting edge 421 is in contact fit with the cutting portion 316. Specifically, the contact fit means that in the process of movement of the blade tube 40 in the axial direction of the sheath 20, the cutting edge 421 and the cutting portion 316 can transition between a separated state and a contact state. When the blade tube 40 moves in the axial direction toward the proximal end, the cutting edge 421 is away and separated from the cutting portion 316. When the blade tube 40 moves in the axial direction toward the distal end, the cutting edge 421 and the cutting portion 316 approach each other and are in abutment contact with each other.

Specifically, as shown in FIG. 4, FIG. 5, and FIG. 12 together, the suture 200 can extend into the sheath 20 through the suture threading channel 301 and is located between the cutting portion 316 and the cutting edge 421. When the blade tube 40 moves along an axial distal end of the sheath 20 relative to the sheath 20, the cutting edge 421 can be in contact with the cutting portion 316 and clamp the suture 200 between the cutting edge 421 and the cutting portion 316.

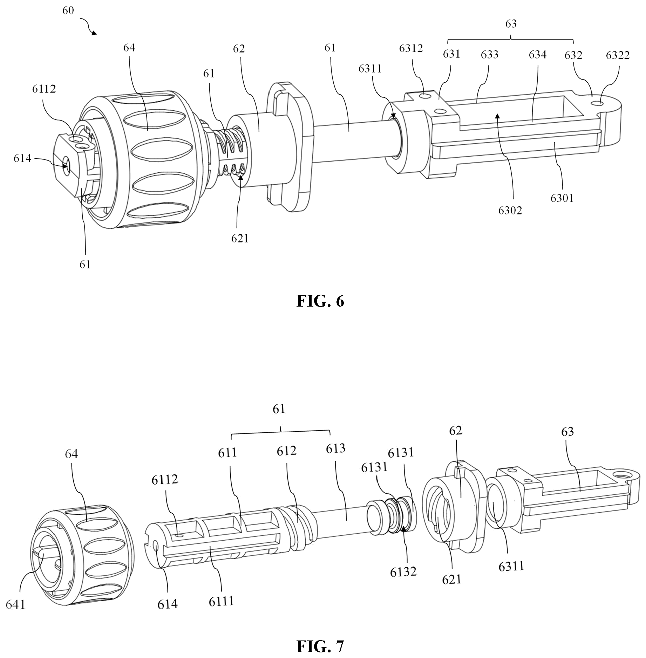

In this embodiment, as shown in FIG. 6, FIG. 8, and FIG. 12, a proximal end of the blade tube 40 is connected to the driving mechanism 60. During movement of the driving mechanism 60 relative to the handle 10, the driving mechanism 60 can at least drive the blade tube 40 to rotate around its own axis, so that the cutting edge 421 is in contact with the cutting portion 316 and clamps the suture 200 between the cutting portion 316 and the cutting edge 421. By driving the blade tube 40 to rotate around its own axis through the driving mechanism 60, the cutting edge 421 rotates relative to the cutting portion 316, thereby allowing the cutting edge 421 to cut the suture 200 in via rotation relative to the cutting portion 316. The cutting edge 421 can simultaneously apply a pressure and a cutting force to the suture 200, thereby enhancing the cutting effect of the cutting edge 421 on the suture 200 and lowering the difficulty in cutting off the suture 200. Moreover, compared with a method for cutting off the suture by pressing, the technical solution provided in the present invention, namely, the method for cutting the suture 200 by rotating the cutting edge 421 relative to the cutting portion 316, requires a low pressure to cut the suture. This can reduce the pushing force applied by the blade tube 40 to the tying head assembly 30 and the pressure between the cutting edge 421 and the cutting portion 316, and reduce the probability of damage to the blade tube 40 or sheath 20.

In some embodiments, as shown in FIG. 4, FIG. 8, and FIG. 9, the suture knot locking system 100 further includes a support sleeve 70. The support sleeve 70 is positioned to surround the outside the push rod 50 and is disposed inside the sheath 20. Specifically, the support sleeve 70 is disposed inside the blade tube 40. The distal end of the support sleeve 70 is connected to the tying head assembly 30, and the proximal end of the support sleeve 70 is connected to the handle 10 for fixation. By adding the support sleeve 70 inside the sheath 20, when the push rod 50 moves toward the distal end and presses the tying head assembly 30, the support sleeve 70 can provide a pulling force toward the proximal end for the tying head assembly 30, to at least counteract part of the pushing force of the push rod 50 toward the distal end. Therefore, the support sleeve 70 at least can help the sheath 20 share part of the pushing force of the push rod 50 toward the distal end, thereby reducing the probability of the sheath 20 being stretched and deformed. This prevents a phenomenon that some components (such as a knot locking member 34 described below) of the tying head assembly 30 cannot fall off from the sheath 20 due to the stretched and deformed sheath 20 when they need to fall off from the sheath 20, and increases the success rate of knot locking. In other embodiments, the support sleeve 70 can also be threaded inside the sheath 20 and disposed outside the blade tube 40, and the blade tube 40 moves in the axial direction within the support sleeve 70. In another embodiment, the distal end of the support sleeve 70 can also be connected to an inner wall of the sheath 20 close to the distal end or the distal end of the sheath 20, which can also reduce the probability of the sheath 20 being stretched and deformed.

In some embodiments, as shown in FIG. 4, FIG. 6, FIG. 7, and FIG. 8, the driving mechanism 60 includes a screw-in member 61 and a moving member 63. Both the screw-in member 61 and the moving member 63 are both movably disposed at the handle 10. The screw-in member 61 can move in the axial direction of the sheath 20 relative to the handle 10 and rotate around the axis of the sheath 20. The proximal end of the blade tube 40 is connected to the screw-in member 61. The blade tube 40 can be driven by the screw-in member 61 to move in the axial direction of the sheath 20 while also rotating around its own axis relative to the sheath 20. In the process of the cutting edge 421 being in contact with the cutting portion 316, the cutting edge 421 can simultaneously apply a pressure and a cutting force to the suture 200 clamped between the cutting edge 421 and the cutting portion 316, thus further lowering the difficulty in cutting off the suture 200.

The moving member 63 can move in the axial direction of the sheath 20 relative to the handle 10, and the proximal end of the push rod 50 or a position close to the proximal end is connected to the moving member 63. The push rod 50 can move in the axial direction under driving of the moving member 63. In this embodiment, the moving member 63 is connected to the proximal end of the screw-in member 61. The screw-in member 61 is provided with a through hole 614 that extends through the proximal end and the distal end of the screw-in member. The proximal end of the blade tube 40 is communicated with the through hole 614. The proximal end of the push rod 50 extends out from the proximal end of the blade tube 40 and is connected to the moving member 63 after passing through the through hole 614. By connecting the moving member 63 to the screw-in member 61, it is convenient for the driving mechanism 60 to simultaneously control the axial pushing of the push rod 50, the axial movement of the blade tube 40, and rotation of the blade tube 40 around its own axis. Therefore, by controlling the driving mechanism 60, the tying head assembly 30 can be controlled to lock the suture 200, and meanwhile, the blade tube 40 can be controlled to cut off the locked suture 200, thus lowering the operation difficulty in performing tying and cutting operations on the suture 200 in the suture knot locking system 100 and reducing operation steps. It is conducive to improving surgical efficiency and shortening operation time. In one embodiment, there is a first setting method: the screw-in member 61 is rotatably connected to the moving member 63, so that the rotation of the screw-in member 61 is not transmitted to the moving member 63; and the movement of the screw-in member 61 can only drive the moving member 63 to do axial movement, thus driving the push rod 50 to axially push the tying head assembly 30. In another embodiment, there is a second setting method: the screw-in member 61 can be fixedly connected to the moving member 63. In this case, the rotation of the screw-in member 61 can be transmitted to the moving member 63, and the movement of the screw-in member 61 causes the moving member 63 to both axially move and rotate, thereby driving the push rod 50 to axially move and rotatably push the tying head assembly 30.

Further, as shown in FIG. 1, FIG. 2, FIG. 3, and FIG. 6, the driving mechanism 60 further includes a fixing member 62. The fixing member 62 is fixedly arranged on the handle 10 and is provided with a threaded hole 621 extending in the axial direction of the sheath 20. The screw-in member 61 is provided with an external thread and is threaded in the threaded hole 621 and in threaded fit with the threaded hole 621. A screw-nut mechanism is formed between the fixing member 62 and the screw-in member 61. When the screw-in member 61 rotates relative to the fixing member 62, the screw-in member 61 can move in the axial direction while rotating around its own axis through the threaded fit between the screw-in member 61 and the fixing member 62. Thus, the screw-in member 61 can enable the blade tube 40 to rotate around its own axis while pushing the blade tube 40 to axially move.

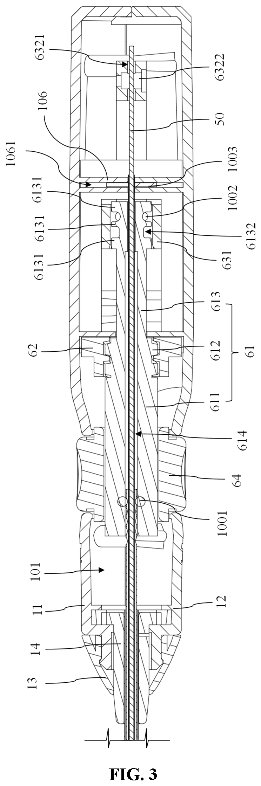

Further, as shown in FIG. 3, FIG. 6, and FIG. 7, the driving mechanism 60 further includes a knob 64. The knob 64 is positioned to surround the outside of the screw-in member 61 and is rotatably arranged on the handle 10. A sliding chute 6111 is formed in one of an inner circumferential surface of the knob 64 and an outer circumferential surface of the screw-in member 61. A sliding block 641 that is in sliding fit with the sliding chute 6111 is arranged on the other one of the inner circumferential surface of the knob and the outer circumferential surface of the screw-in member. The sliding chute 6111 extends in the axial direction of the sheath 20. It can be understood that through the fit between the sliding chute 6111 and the sliding block 641, the knob 64 and the screw-in member 61 can move relative to each other in the axial direction of the sheath 20. However, the fit between the sliding chute 6111 and the sliding block 641 limits the relative rotation between the knob 64 and the screw-in member 61 around their own axes. Therefore, when the knob 64 is driven to rotate, through the fit between the sliding chute 6111 and the sliding block 641, the knob 64 can drive the screw-in member 61 to rotate around its own axis relative to the handle 10 and the fixing member 62. Under the action of the threaded fit between the screw-in member 61 and the fixing member 62, the screw-in member 61 simultaneously moves in the axial direction of the sheath 20 relative to the handle 10 and the fixing member 62. In this implementation, the knob 64 is controlled to perform one-click control on the axial pushing of the push rod 50, the axial movement of the blade tube 40, and rotation of the blade tube 40 around its own axis. Therefore, by controlling the knob 64, the tying head assembly 30 can be controlled to lock the suture 200, and meanwhile, the blade tube 40 can be controlled to cut off the locked suture 200, thus lowering the operation difficulty in performing tying and cutting operations on the suture 200 in the suture knot locking system 100 and reducing operation steps. It is conducive to improving surgical efficiency and shortening operation time.

In some embodiments of the present invention, the tying head assembly 30 includes a tying head seat 31 and a knot locking member 34. The tying head seat 31 is threaded in the sheath 20 and is fixedly connected to the sheath 20. The tying head seat 31 is provided with a seat hole 315 that extends through the proximal end and the distal end of the tying head seat 31. The knot locking member 34 is detachably connected to the tying head seat 31 within the seat hole 315, and is configured to lock the suture 200.

In some embodiments of the present invention, as shown in FIG. 2, FIG. 4, and FIG. 14, the knot locking member 34 includes a tying head 32 and a tying pin 33. The tying head 32 is clamped in the seat hole 315. The tying head 32 is provided with a pin hole 321 extending through the proximal end and the distal end of the tying head 32. The pin hole 321 and the seat hole 315 are communicated with each other and jointly define a suture threading channel 301. The distal end of the support sleeve 70 is connected to the tying head seat 31 and is communicated with the pin hole 321. When the tying head assembly 30 is in an initial state, the tying pin 33 is disposed inside the support sleeve 70 and is located between the tying head 32 and the push rod 50, and the tying pin 33 can move within the support sleeve 70 under the pushing of the push rod 50. Specifically, when the push rod 50 moves toward the distal end, the push rod 50 pushes the tying pin 33 to move within the support sleeve 70 toward the distal end. With the continuous pushing performed by the push rod 50, the tying pin 33 can be inserted into the pin hole 321, and is in inserted connection with the tying head 32, thereby locking the suture threaded in the pin hole 321 and causing the tying head assembly 30 to transition into a locked state.

It should be noted that the maximum outer diameter of the tying pin 33 is greater than the inner diameter of the pin hole 321. When the tying pin 33 is inserted into the pin hole 321, the tying head 32 at least partially deforms to implement firmer inserted connection between the tying pin 33 and the tying head 32. This reduces the probability of the tying pin 33 falling off from the pin hole 321 and improving the firmness of tying the suture 200 by the tying head assembly 30. In another embodiment, the tying pin 33 at least partially undergoes compression deformation to ensure the firmer inserted connection with the tying head 32. In other embodiments, the tying head seat 31 may not be provided, and the tying head 32 is directly connected to the sheath 20 in a close fit manner. By setting the force between the tying pin 33 and the tying head 32 to be less than the force between the tying head 32 and the sheath 20, the tying head 32 cannot be pushed out of the sheath when the tying pin 33 is inserted into the pin hole 321 of the tying head 32. For example, this is implemented by configuring friction surfaces with different friction coefficients between the tying pin 33, the tying head 32, and the sheath 20.

In this embodiment, specifically when the tying head assembly 30 includes the tying head 32 and the tying pin 33, the screw-in member 61 is rotatably connected to the moving member 63, so that the rotation of the screw-in member 61 is not transmitted to the moving member 63, and the moving member 63 only moves axially, thereby driving the push rod 50 to axially push the tying pin 33 to be inserted into the tying head 32. In other embodiments, the screw-in member 61 can be fixedly connected to the moving member 63, so that the rotation of the screw-in member 61 can be transmitted to the moving member 63, and the movement of the screw-in member 61 causes the moving member 63 to both axially move and rotate, thereby driving the push rod 50 to axially move and rotatably push the tying pin 33 to be inserted into the tying head 32. In other embodiments, the knot locking member 34 can always be in the automatic knot locking state. For example, there is a channel capable of being automatically locked inside the knot locking member 34, and the inner diameter of the channel is less than the outer diameter of the suture 200. The interior of the knot locking member 34 is made of an elastic material. The suture 200 is threaded into the channel. The suture 200 and the knot locking member 34 can be fixed to each other without an external force.

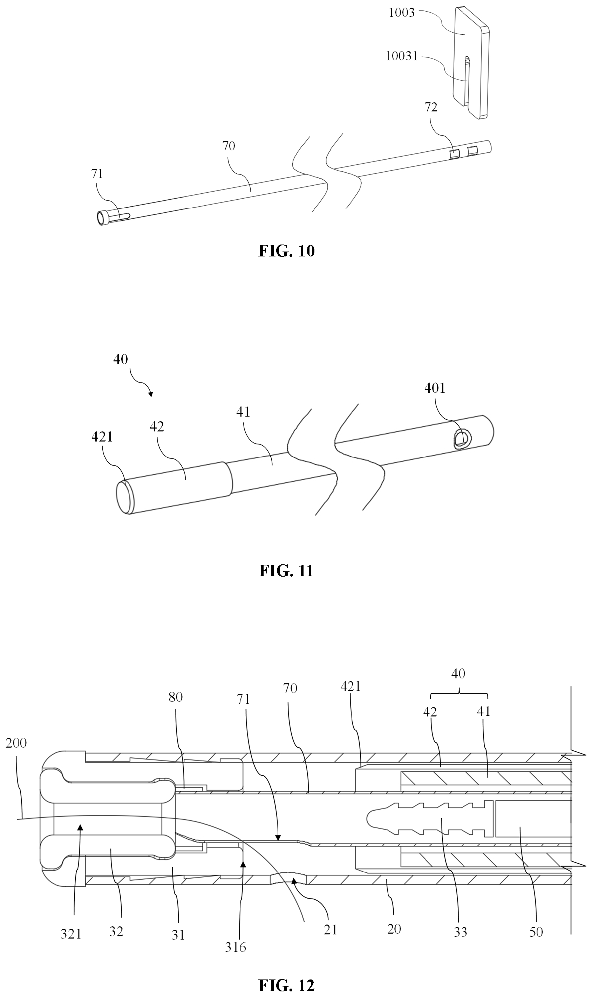

In some embodiments of the present invention, as shown in FIG. 4 and FIG. 12, the suture knot locking system 100 further includes a connection sleeve 80. The connection sleeve 80 is positioned to surround the outside of the support sleeve 70 and is located inside the seat hole 315. Two sides of the connection sleeve 80 are respectively fixedly connected to the support sleeve 70 and the tying head seat 31. By the arrangement of the connection sleeve 80, the distal end of the support sleeve 70 surrounds the connection sleeve 80 and is then connected to the tying head seat 31 through the connection sleeve 80. The tying head seat 31 is then connected to the sheath 20. The sheath 20, the tying head seat 31, the connection sleeve 80, and the support sleeve 70 can be connected to each other by adding glue, to keep them relatively fixed. This improves firmness of connection between the connection sleeve 80 and the support sleeve 70, tying head seat 31, as well as sheath 20, so that the support sleeve 70 can at least partially counteract the pushing force of the push rod 50 toward the distal end, and the support sleeve 70 can better help the sheath 20 share the pushing force of the push rod 50 toward the distal end, thereby reducing the probability of the sheath 20 being stretched and deformed. This prevents a phenomenon that some components (such as a knot locking member 34 described below) of the tying head assembly 30 cannot fall off from the sheath 20 due to the stretched and deformed sheath 20 when they need to fall off from the sheath 20, and the increases the success rate of knot locking. The sheath 20, the tying head seat 31, the connection sleeve 80, and the support sleeve 70 are connected to each other through glue or by welding.

Further, as shown in FIG. 4 and FIG. 5, a ringlike slot 3154 extending in a circumferential direction of the seat hole 315 is formed inside the seat hole 315, and the connection sleeve 80 is arranged in the ringlike slot 3154. By the formation of the ringlike slot 3154 inside the seat hole 315, when the pushing force of the push rod 50 is transmitted to the tying head seat 31, the proximal end of the connection sleeve 80 can resist against a side wall of the ringlike slot 3154, to reduce the probability of the connection sleeve 80 being detached from the seat hole 315 under the action of the pulling force of the support sleeve 70.

In some embodiments of the present invention, as shown in FIG. 10 and FIG. 12, a first suture passing hole 71 is formed in an outer circumferential surface of the support sleeve 70. A second suture passing hole 21 is formed in an outer circumferential surface of the sheath 20. The suture 200 can extend into the support sleeve 70 via the suture threading channel 301 and extend out of the sheath 20 sequentially through the first suture passing hole 71 and the second suture passing hole 21, so that the suture 200 is at least partially located between the cutting portion 316 and the cutting edge 421. When the blade tube 40 moves toward the distal end under the driving of the driving mechanism 60, the suture 200 can be clamped between the cutting edge 421 and the cutting portion 316 and is cut off. In one embodiment, only the first suture passing hole 71 in the support sleeve 70 can be provided. For example, after the suture 200 is threaded into the first suture passing hole 71, the suture 200 can extend along an inner cavity of the sheath 20 to the proximal end of the sheath 20. In this case, the suture 200 is at least partially located between the cutting portion 316 and the cutting edge 421. When the blade tube 40 moves toward the distal end under the driving of the driving mechanism 60, the suture 200 can be clamped between the cutting edge 421 and the cutting portion 316 and is cut off. In one embodiment, only the second suture passing hole 21 in the outer circumferential surface of the sheath 20 may be provided. The suture 200 does not pass through the support sleeve 70 in the process of entering the position between the cutting portion 316 and the cutting edge 421 through the pin hole 321, but bypasses the distal end of the support sleeve 70. In this case, the support sleeve 70 can be be positioned to surround the outside of the blade tube 40.

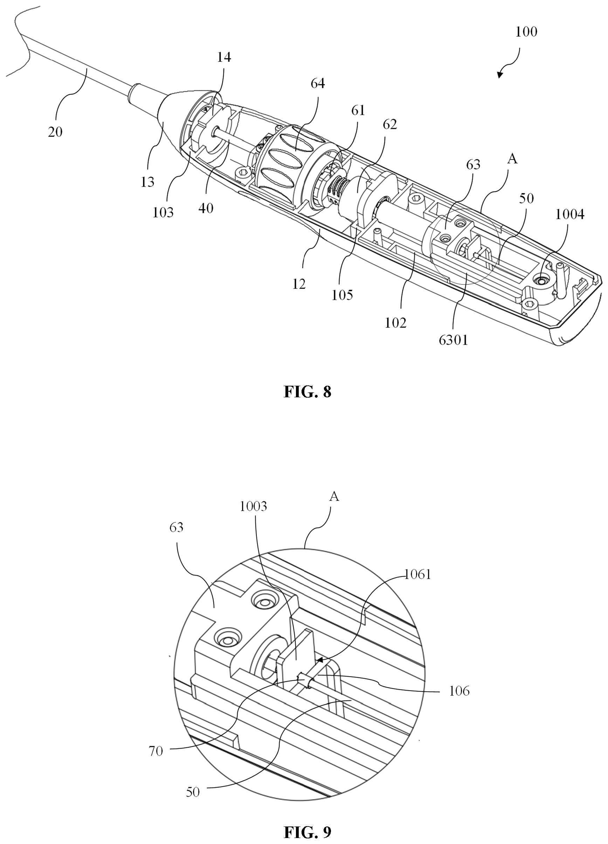

Referring to FIG. 2, FIG. 3, FIG. 6, and FIG. 8, the push rod 50, the support sleeve 70, the blade tube 40, and the sheath 20 are sequentially nested from inside to outside. Furthermore, both the sheath 20 and the support sleeve 70 are fixedly connected to the handle 10, while the proximal ends of the push rod 50 and the blade tube 40 are connected to the driving mechanism 60 and can respectively move in the axial direction of the sheath 20 relative to the sheath 20 and support sleeve 70, so as to achieve the above functions and prevent mutual interference between the push rod 50, the support sleeve 70, the blade tube 40, and the sheath 20. In some embodiments of the present invention, the following solutions are provided:
Further, referring to FIG. 3, the handle 10 is provided with an accommodating cavity 101. The proximal end of the sheath 20 is connected to the distal end of the handle 10 and is communicated with the accommodating cavity 101. In the direction from the distal end to the proximal end, as shown in FIG. 8 and FIG. 9, a second fixing slot 105 and a support portion 106 are sequentially arranged on an inner wall of the accommodating cavity 101. The second fixing slot 105 and the support portion 106 are both located inside the accommodating cavity 101, and the fixing member 62 is fixedly mounted in the second fixing slot 105. The proximal end of the blade tube 40 extends out from the proximal end of the sheath 20 into the accommodating cavity 101, is inserted into the through hole 614 of the screw-in member 61, and is fixedly connected to the screw-in member 61. The proximal end of the screw-in member 61 is rotatably connected to the moving member 63. One of the moving member 63 and the handle 10 is provided with a first sliding structure 102 extending in the axial direction of the sheath 20, and the other one is provided with a second sliding structure 6301 that is slidably connected to the first sliding structure 102, so that the screw-in member 61 can drive the moving member 63 to move in the axial direction of the support sleeve 70 and the moving member 63 cannot rotate together with the screw-in member 61 under the restriction of the first sliding structure 102 and the second sliding structure 6301. The moving member 63 is provided with an avoidance slot 6302. A proximal end of the through hole 614 is communicated with the avoidance slot 6302. The support portion 106 protrudes in a radial direction of the sheath 20 and extends into the avoidance slot 6302. The proximal end of the support sleeve 70 extends from the proximal end of the through hole 614 into the avoidance slot 6302 and is connected to the support portion 106. The proximal end of the push rod 50 extends out from the proximal end of the support sleeve 70 and is connected to the moving member 63.

In the embodiments of the present invention, the sheath 20 is coaxial with the blade tube 40.

The technical solutions of the present invention will be further explained in detail below with specific embodiments.

### Embodiment 1

In this embodiment, as shown in FIG. 1, FIG. 2, FIG. 4, and FIG. 6, the suture knot locking system 100 includes: a handle 10, a sheath 20, a tying head assembly 30, a blade tube 40, a push rod 50, a support sleeve 70, a connection sleeve 80, and a driving mechanism 60.

Specifically, as shown in FIG. 2 and FIG. 3, the handle 10 includes an upper shell 11, a lower shell 12, an end cover 13, and a stress relief sleeve 14. The upper shell 11 and the lower shell 12 are arranged in an oppositely folded manner. An accommodating cavity 101 is defined between the upper shell 11 and the lower shell 12. The end cover 13 has a conical housing. The stress relief sleeve 14 includes a fixing portion 141 and a tubular portion 142. The tubular portion 142 is an axially extending tubular structure, and the fixing portion 141 is disposed at a proximal end of the tubular portion 142. A first fixing slot 103 for mounting the fixing portion 141 and an end cover mounting portion 104 located on one side of the distal end of the first fixing slot 103 are provided at distal ends of the upper shell 11 and the lower shell 12. The end cover 13 is mounted at the end cover mounting portion 104. The fixing portion 141 is mounted in the first fixing slot 103. The distal end of the tubular part 142 sequentially passes through the end cover mounting portion 104 and the end cover 13 and extends out from the distal end of the end cover 13. The proximal end of the sheath 20 is inserted into the tubular portion 142 of the stress relief sleeve 14 and is fixedly connected to the tubular portion 142, and the proximal end of the sheath 20 is communicated with the accommodating cavity 101.

As shown in FIG. 2 and FIG. 4, the tying head assembly 30 includes a tying head seat 31, a tying head 32, and a tying pin 33. The tying head seat 31 is threaded in the sheath 20 and is fixedly connected to the sheath 20.

Specifically, as shown in FIG. 4 and FIG. 5, in an axial direction of the sheath 20, an outer circumferential surface of the tying head seat 31 has a stepped configuration. The tying head seat 31 includes a first stepped section 311, a second stepped section 312, and a third stepped section 313 that are connected in sequence. The first stepped section 311 is located at the distal end, and the third stepped section 313 is located at the proximal end. The outer diameter of the first stepped section 311 is greater than the outer diameter of the third stepped section 313, and the outer diameter of the third stepped section 313 is greater than the outer diameter of the second stepped section 312. The sheath 20 surrounds the outside of the second stepped section 312 and the third stepped section 313. A step surface 314 facing the proximal end is formed between the first stepped section 311 and the second stepped section 312. The distal end of the sheath 20 abuts against the step surface 314, and a gap is formed between an inner wall surface of the sheath 20 and an outer circumferential surface of the second stepped section 312. This gap is configured to accommodate glue. After the glue is cured, it enhances the strength of connection between the sheath 20 and the tying head seat 31.

The tying head seat 31 is provided with a seat hole 315 extending through the proximal end and the distal end of the tying head seat 31. A clamping slot 3153 is further provided in the seat hole 315. The clamping slot 3153 is ringlike and extends in a circumferential direction of the seat hole 315. A ringlike protrusion 322 that is in clamping fit with the clamping slot 3153 is arranged on an outer circumferential surface of the proximal end of the tying head 32. Through the fit between the ringlike protrusion 322 and the clamping slot 3153, the tying head 32 is in clamped connection to the tying head seat 31. When the tying head 32 undergoes a pushing force toward the distal end, and the pushing force is greater than the clamping force between the ringlike protrusion 322 and the clamping slot 3153, the tying head 32 can fall off from the tying head seat 31. To ensure the tying head 32 falls off smoothly, the distal end of the ringlike protrusion 322 and the side wall of the distal end of the clamping slot 3153 are both configured as inclined surfaces.

Further, a stop portion 323 is convexly arranged on an outer circumferential surface of the distal end of the tying head 32. A stop slot 317 is provided in an end surface of the distal end of the tying head seat 31. The end surface of the proximal end of the stop portion 323 provides an abutment fit with the side wall of the proximal end of the stop slot 317, to prevent the tying head 32 from being too deep into the tying head seat 31 when the tying head 32 is assembled into the tying head seat 31.

The tying head 32 is provided with a pin hole 321 extending through the proximal end and the distal end of the tying head 32. The pin hole 321 and the seat hole 315 are communicated with each other and jointly define a suture threading channel 301. A ringlike slot 3154 extending in a circumferential direction of the seat hole 315 is formed in the seat hole 315. The ringlike slot 3154 is located on one side of the proximal end of the clamping slot 3153. The connection sleeve 80 is disposed in the ringlike slot 3154 and surrounds the outside of the support sleeve 70. The sheath 20, the tying head seat 31, the connection sleeve 80, and the support sleeve 70 are adhered and fixed together by glue. The distal end of the support sleeve 70 resists against the proximal end of the tying head 32 and is communicated with the pin hole 321. The push rod 50 is disposed in the support sleeve 70 and can move axially relative to the support sleeve 70. The tying pin 33 is disposed in the support sleeve 70 and is located between the tying head 32 and the distal end of the push rod 50. The tying pin 33 can move within the support sleeve 70 under the pushing of the push rod 50. When the push rod 50 moves toward the distal end, the push rod 50 pushes the tying pin 33 to move within the support sleeve 70 toward the distal end. With the continuous pushing performed by the push rod 50, the tying pin 33 can be inserted into the pin hole 321, and is in inserted connection with the tying head 32, thereby locking the suture threaded in the pin hole 321.

In this embodiment, as shown in FIG. 4 and FIG. 5, the proximal end of the pin hole 321 has a trumpet-shaped flared structure to facilitate guidance for insertion of the tying pin 33 into the pin hole 321. The tying pin 33 includes a cylindrical main body portion 331, a limiting portion 332 disposed at the proximal end of the main body portion 331, and a raised portion 333 annularly arranged on an outer circumferential surface of the main body portion 331. The distal end of the main body portion 331 is configured as a smooth transition arc-shaped surface, which has a guide effect during the insertion of the tying pin 33 into the pin hole 321. An end surface of the distal end of the raised portion 333 is configured as an inclined surface, and the maximum outer diameter of the raised portion 333 is greater than the inner diameter of the pin hole 321. When the main body portion 331 of the tying pin 33 is inserted into the pin hole 321, the raised portion 333 undergoes elastic deformation and is radially compressed. The elastic force generated by the deformation of the raised portion 333 is used to firmly fix the tying pin 33 into the pin hole 321 and lock the suture threaded in the pin hole 321. The outer diameter of the limiting portion 332 is greater than the outer diameter of the pin hole 321, so that an end surface of the distal end of the limiting portion 332 resists against an end surface of the proximal end of the tying head 32 to limit the depth of insertion of the tying pin 33 into the tying head 32. When the push rod 50 continues to move toward the distal end, the limiting portion 332 pushes the tying head 32 out of the seat hole 315. In another embodiment, between the tying pin 33 and the tying head 32, the tying head 32 easily deforms. The outer diameter of the tying pin 33 is greater than the inner diameter of the pin hole 321 of the tying head 32. When the tying pin 33 is inserted into the tying head 32, the tying head 32 can undergo expansion deformation, and the inner diameter of the pin hole 321 can be enlarged by the tying pin 33, so that the tying pin 33 can be firmly fixed in the pin hole 321.

Further, the blade tube 40 surrounds the outside of the support sleeve 70 and is located inside the sheath 20. The blade tube 40 can move relative to the sheath 20 in the axial direction of the sheath 20. A cutting edge 421 is disposed at the distal end of the blade tube 40. A cutting portion 316 is disposed at the proximal end of the tying head seat 31. The cutting portion 316 is specifically a cutting surface formed on the end surface of the proximal end of the tying head seat 31. The cutting edge 421 is opposite to the cutting portion 316 in the axial direction of the sheath 20. When the blade tube 40 moves axially, the cutting edge 421 and the cutting portion 316 can transition between a separated state and a resisting contact state.

As shown in FIG. 12, a first suture passing hole 71 is formed in an outer circumferential surface of the support sleeve 70. A second suture passing hole 21 is formed in an outer circumferential surface of the sheath 20. The suture 200 can extend into the support sleeve 70 via the suture threading channel 301 and extend out of the sheath 20 sequentially through the first suture passing hole 71 and the second suture passing hole 21, so that the suture 200 is at least partially located between the cutting portion 316 and the cutting edge 421. When the blade tube 40 moves toward the distal end, the suture 200 can be clamped between the cutting edge 421 and the cutting portion 316 and is cut off.

In this embodiment, as shown in FIG. 2, FIG. 6, FIG. 7, and FIG. 8, the driving mechanism 60 includes a screw-in member 61, a fixing member 62, a moving member 63, and a knob 64. In a direction from the distal end to the proximal end, a second fixing slot 105 and a support portion 106 that are spaced apart are sequentially disposed on an inner wall of the accommodating cavity 101. The fixing member 62 is fixedly mounted in the second fixing slot 105, and is provided with a threaded hole 621 that extends in the axial direction of the sheath 20 and extends through the proximal end and the distal end of the fixing member 62. The entire screw-in member 61 has a long-strip shape that extends in the axial direction. In a direction from the proximal end to the distal end, the screw-in member 61 includes a slidable connection portion 611, a threaded fit portion 612, and a connection rod portion 613 that are sequentially connected. An external thread that is in threaded fit with the threaded hole 621 is provided on an outer circumferential surface of the threaded fit portion 612. The threaded fit portion 612 of the screw-in member 61 is threaded in the threaded hole 621 of the fixing member 62 and is in threaded fit with the threaded hole 621. The slidable connection portion 611 is located on one side of the distal end of the fixing member 62. The connection rod portion 613 is located on one side of a proximal end of the fixing member 62. A sliding chute 6112 extending in the axial direction of the sheath 20 is formed in an outer circumferential surface of the slidable connection portion 611. The knob 64 is tubular and surrounds the outside of the slidable connection portion 611. A sliding block 641 extending in the axial direction of the sheath 20 is provided on an inner wall surface of the knob 64. The sliding block 641 is slidably disposed in the sliding chute 6111 and is rotatably disposed on the handle 10 through the knob 64. When the knob 64 is driven to rotate, through the fit between the sliding chute 6111 and the sliding block 641, the knob 64 can drive the screw-in member 61 to rotate around its own axis relative to the handle 10 and the fixing member 62. Under the action of the threaded fit between the screw-in member 61 and the fixing member 62, the screw-in member 61 simultaneously moves in the axial direction of the sheath 20 relative to the handle 10 and the fixing member 62.

Further, as shown in FIG. 3, FIG. 6, FIG. 7, and FIG. 11, the screw-in member 61 is provided with a through hole 614 extending through the proximal end and the distal end of the screw-in member 61. The slidable connection portion 611 is further provided with a plurality of first pin shaft holes 6112 arranged in the radial direction of the sheath 20. The first pin shaft holes 6112 are communicated with the through hole 614. A first snap-in slot 401 is formed in an outer circumferential surface of the blade tube 40. The first snap-in slot 401 is close to the proximal end of the blade tube 40. The proximal end of the blade tube 40 is inserted into the through hole 614, and a first pin shaft 1001 is inserted into the first pin shaft hole 6112 to cause a portion of the pin shaft extending into the through hole 614 to be snapped in the first snap-in slot 401, so that the proximal end of the blade tube 40 is fixedly connected to the screw-in member 61.

In some embodiments, two of a plurality of first snap-in slots 401 form a group. A plurality of groups of first snap-in slots 401 are spaced apart sequentially in the axial direction of the blade tube 40. The two first snap-in slots 401 in each group are respectively located on left and right sides of an axis of the blade tube 40. Two of a plurality of first pin shaft holes 6112 form a group, and a plurality of groups of first pin shaft holes 6112 are spaced apart sequentially in the axial direction of the blade tube 40. The two first pin shaft holes 6112 in each group are respectively located on left and right sides of an axis of the through hole 614. The two first pin shaft holes 6112 in each group can be aligned with the two first snap-in slots 401 in each group, so that two first pin shafts 1001 embedded into the first pin shaft holes 6112 can be respectively snapped in the two first snap-in slots 401. It should be noted that by the arrangement of the plurality of groups of first snap-in slots 401 and the plurality of groups of first pin shaft holes 6112 in the axial direction, the firmness of the connection between the blade tube 40 and the screw-in member 61 can be improved. Furthermore, the first snap-in slots 401 are aligned with the first pin shaft holes 6112 in different groups according to the need to adjust the relative position between the blade tube 40 and the tying head assembly 30 in the axial direction and then adjust the relative position between the distal end of the blade tube 40 and the distal end of the push rod 50 to ensure that the cutting edge 421 at the distal end of the blade tube 40 can be in contact with the cutting portion 316 and cut off the suture 200 after the tying pin 33 is fully inserted into the tying head 32. In other embodiments, there may be one first snap-in slot 401.

Further, as shown in FIG. 6 and FIG. 7, the moving member 63 includes a rotatable connection portion 631 and a fixed connection portion 632 that are spaced apart in the axial direction of the sheath 20. The moving member 63 further includes a first side wall 633 and a second side wall 634 that are connected to the rotatable connection portion 631 and the fixed connection portion 632. The first side wall 633 and the second side wall 634 are spaced apart from each other in the radial direction of the sheath 20. The rotatable connection portion 631, the first side wall 633, the fixed connection portion 632, and the second side wall 634 enclose an avoidance slot 6302. The rotatable connection portion 631 is provided with a rotatable limiting hole 6311 extending through the proximal end and the distal end of the rotatable connection portion 631. The rotatable connection portion 631 is further provided with a second pin shaft hole 6312 extending in the radial direction of the sheath 20, and the second pin shaft hole 6312 is communicated with the rotatable limiting hole 6311.

Referring to FIG. 3, FIG. 6, and FIG. 7, a plurality of rotatable limiting portions 6131 that are spaced apart sequentially in the axial direction of the sheath are provided on the proximal end of the connection rod portion 613 of the screw-in member 61. The rotatable limiting portions 6131 are ringlike and protrude out of an outer circumferential surface of the connection rod portion 613. A limiting gap 6132 is formed between two adjacent rotatable limiting portions 6131. The distal end of the connection rod portion 613 is rotatably inserted into the rotatable limiting hole 6311. A second pin shaft 1002 is embedded into the second pin shaft hole 6312, and a portion of the second pin shaft 1002 located in the rotatable limiting hole 6311 is disposed in the limiting gap 6132, so that in the axial direction of the sheath 20, the second pin shaft 1002 is respectively in stop fit with two adjacent rotatable limiting portions 6131. Thus, the screw-in member 61 can rotate around the axis of the blade tube 40 relative to the moving member 63, but the stop fit between the second pin shaft 1002 and the rotatable limiting portions 6131 restrains relative movement between the screw-in member 61 and the moving member 63 in the axial direction of the sheath 20.

As shown in FIG. 8, FIG. 9, and FIG. 10, the support portion 106 includes a plurality of platelike side plate portions arranged in the radial direction of the sheath 20, and an insertion slot 1061 with an opening in one side is formed between the plurality of side plate portions. A second snap-in slot 72 is formed in the outer circumferential surface of the support sleeve 70. The proximal end of the support sleeve 70 extends out from the proximal end of the blade tube 40 and extends into the avoidance slot 6302 after passing through the through hole 614. The suture knot locking system 100 further includes a tube clamp 1003. The tube clamp 1003 is provided with a third snap-in slot 10031 with an opening in one end. The tube clamp 1003 is inserted into the insertion slot 1061 and is snapped in the second snap-in slot 72 of the support sleeve 70, so that the tube clamp 1003 is respectively in stop fit with a side wall of a proximal end of the second snap-in slot 72 and a side wall of a distal end of the second snap-in slot 72, thus restraining movement of the support sleeve 70 in the axial direction of the sheath 20 relative to the handle 10.

In some embodiments, as shown in FIG. 10, a plurality of second snap-in slots 72 are formed in the outer circumferential surface of the support sleeve 70. Two of the plurality of second snap-in slots 72 form a group. A plurality of groups of second snap-in slots 72 are spaced apart sequentially in the axial direction of the support sleeve 70. The two second snap-in slots 72 in each group are respectively located on left and right sides of the axis of the support sleeve 70. The support portion 106 is provided with a plurality of insertion slots 1061. Each insertion slot 1061 is provided with a tube clamp 1003 and corresponds to one second snap-in slot 72, to enhance the strength of the connection between the support sleeve 70 and the support portion 106. In other embodiments, there may be one second snap-in slot 72.

Further, as shown in FIG. 3, FIG. 6, and FIG. 8, the fixed connection portion 632 is provided with a first fastening hole 6321 extending in the axial direction and a second fastening hole 6322 extending in the radial direction of the sheath 20. The second fastening hole 6322 is configured as a threaded hole and is communicated with the first fastening hole 6321. A fastening screw 1004 is disposed in the second fastening hole 6322. The proximal end of the push rod 50 is threaded in the first fastening hole 6321, and a fastening screw 1004 passing through the second fastening hole 6322 locks and fixes the push rod 50 to the fixed connection portion 632.

Further, as shown in FIG. 8, one of the moving member 63 and the handle 10 is provided with a first sliding structure 102 extending in the axial direction of the sheath 20, and the other one is provided with a second sliding structure 6301 that is slidably connected to the first sliding structure 102, so that the screw-in member 61 can drive the moving member 63 to move in the axial direction of the support sleeve 70 and the moving member 63 cannot rotate together with the screw-in member 61 under the restriction of the first sliding structure 102 and the second sliding structure 6301.

It can be understood that the first sliding structure 102 and the second sliding structure 6301 can be configured in various structural forms of sliding fit. For example, the first sliding structure 102 is a sliding chute, and the second sliding structure 6301 is a sliding block that is in sliding fit with the sliding chute. For another example, the first sliding structure 102 is configured as a sliding rod, and the second sliding structure 6301 is configured as a sliding hole that is in sliding fit with the sliding rod.

In this embodiment, the knob 64 is controlled to perform one-click control on the axial pushing of the push rod 50, the axial movement of the blade tube 40, and rotation of the blade tube 40 around its own axis. Therefore, by controlling the knob 64, the tying head assembly 30 can be controlled to lock the suture 200, and meanwhile, the blade tube 40 can be controlled to cut off the locked suture 200, thus lowering the operation difficulty in performing tying and cutting operations on the suture 200 in the suture knot locking system 100 and reducing operation steps. It is conducive to improving surgical efficiency and shortening operation time. It should be also noted that by adding the support sleeve 70, the pushing force of the push rod 50 toward the distal end can be at least partially counteracted by the support sleeve 70, so that the support sleeve 70 can better help the sheath 20 share the pushing force of the push rod 50 toward the distal end, thereby reducing the probability of the sheath 20 being stretched and deformed.

In some embodiments of the present invention, as shown in FIG. 1, FIG. 2, FIG. 12, and FIG. 14, the suture knot locking system 100 further includes a suture guider 90 which includes a suture guide grip 91 and a suture guide wire 92 connected to the suture guide grip 91. The distal end of the suture guide wire 92 can sequentially extend into the support sleeve 70 through the second suture passing hole 21 and the first suture passing hole 71, and is then threaded out from the tying head 32 via the seat hole 315 of the tying head seat 31 and the pin hole 321 of the tying head 32. The suture guide wire 92 is an annular sleeve structure formed by folding a wire. The suture guide grip 91 is approximately in a flat plate shape. Plate surfaces of two sides of the suture guide grip 91 are recessed inwards and are provided with a plurality of long-strip-shaped protrusions extending along the plate surfaces, to increase friction and facilitate holding. Two suspended arm portions 911 that are spaced apart from each other are arranged at one end of the suture guide grip 91. The two suspended arm portions 911 are configured to clamp the suture guide grip 91 outside the sheath 20 and can slide in the axial direction of the sheath 20 relative to the sheath 20.

In this embodiment, operations in a suture knot locking process of the suture knot locking system 100 are as follows:
Patent foramen ovale suture closure is taken as an example. After the patent foramen ovale suture closure is completed, an end portion of the suture 200 is exposed outside the body. First, the suture guide wire 92 is used to capture the exposed portion of the suture 200 that is exposed outside the body. The suture guide grip 91 is controlled to slide toward one side of the proximal end in the axial direction of the sheath 20, so that the suture guide wire 92 pulls the exposed portion of the suture 200 to sequentially pass through the pin hole 321 and the seat hole 315 and enter the support sleeve 70, and the exposed portion is then threaded out of the sheath 20 via the first suture passing hole 71 and the second suture passing hole 21. Then, by controlling the suture knot locking system 100, the distal end of the sheath 20 is pushed to a suturing position under the guidance of the suture 200, and the knob 64 is driven to rotate. The knob 64 drives the push rod 50 and the blade tube 40 to be simultaneously pushed toward the distal end. Under the continuous pushing of the push rod 50, the tying pin 33 can be inserted into the pin hole 321, so that the tying pin 33 and the tying head 32 are in insertion connection with each other into a whole, and then the suture threaded in the pin hole 321 is locked. The cutting edge 421 of the blade tube 40 moves toward the distal end to resist against the cutting portion 316 of the tying head seat 31. Furthermore, under the driving of the knob 64, the blade tube 40 rotates around its own axis while moving toward the distal end, so that the cutting edge 421 applies a pressure and a cutting force to the suture 200 clamped between the cutting edge 421 and the cutting portion 316, to cut off the suture 200. Finally, the knob 64 is continued to be driven to rotate, and the push rod 50 continues to move toward the distal end until the tying pin 33 and the tying head 32 are pushed out from the seat hole 315 of the tying head seat 31, thus completing the locking on the suture 200. The sheath 20 and the tying head seat 31 are withdrawn from the body, thus completing the knot locking operation on the suture 200.

In some other embodiments of the present invention, as shown in FIG. 13, the cutting portion 316 is disposed on an inner wall surface of the suture threading channel 301. The cutting edge 421 is formed on the outer circumferential surface of the blade tube 40. The outer circumferential surface of the cutting edge 421 is in contact fit with an inner ring surface of the cutting portion 316. In this embodiment, the seat hole 315 of the tying head seat 31 is stepped. The seat hole 315 includes a first stepped segment 3151 and a second stepped segment 3152 that are communicated with each other. The first stepped segment 3151 is located at the proximal end relative to the second stepped segment 3152, and the inner diameter of the first stepped segment 3151 is greater than the inner diameter of the second stepped segment 3152. The cutting portion 316 is formed on an inner wall of the first stepped segment 3151. The distal end of the blade tube 40 can be inserted into the seat hole 315 of the first stepped segment 3151. The cutting edge 421 is formed on the outer circumferential surface of the blade tube 40 and can be in contact fit with the inner wall of the first stepped segment 3151. In the process of controlling the knob 64 to control the axial movement of the blade tube 40 and the rotation of the blade tube 40 around its own axis, the cutting edge 421 continuously cuts the suture 200 located between the cutting edge 421 and the cutting portion 316 during the rotation, thus cutting off the suture 200.

### Embodiment 2

A difference between Embodiment 2 and Embodiment 1 will be described below, and the same or similar parts in Embodiment 2 and Embodiment 1 will not be elaborated here.

In this embodiment, as shown in FIG. 11 and FIG. 12, the blade tube 40 includes a main body section 41 and a cutting section 42 connected to the distal end of the main body section 41. The cutting edge 421 is disposed at the distal end of the cutting section 42. Bending resistance of the cutting section 42 is higher than bending resistance of the main body section 41.

Specifically, the main body section 41 has higher bending performance than the cutting section 42, so that the main body section 41 can comply with a curved structure of a blood vessel in a human body. It is conducive to more smoothly placing the main body section 41 to a target position in the human body along with the intervention of the sheath 20. The cutting section 42 has higher hardness, rigidity, and bending resistance than the main body section 41. In the process of pushing the cutting edge 421 at the distal end of the blade tube 40 and performing a cutting action, the cutting section 42 can apply sufficient pressure and cutting force to the suture 200 between the cutting edge 421 and the cutting portion 316, to ensure that the cutting edge 421 cuts off the suture 200.

In some embodiments, the cutting section 42 and the main body section 41 are made of the same materials, but the wall thickness of the main body section 41 is less than the wall thickness of the cutting section 42, so that the bending performance of the main body section 41 is higher than the bending performance of the cutting section 42, namely, the main body section 41 has higher flexibility than the cutting section 42.

In some embodiments, as shown in FIG. 12, the cutting section 42 and the main body section 41 are made of different materials, and the hardness and rigidity of the material of the cutting section 42 are higher than the hardness and rigidity of the material of the main body section 41. For example, the cutting section 42 can be made of a steel material, or the like. The supporting performance of the cutting section 42 is higher than the supporting performance of the main body section 41. The cutting section 42 at least partially surrounds the outside of the main body section 41. The cutting section 42 and the main body section 41 are connected into an integrated structure by laser welding, soldering, or gluing.

The foregoing descriptions are merely preferred specific embodiments of the present invention, but are not intended to limit the protection scope of the present invention. Any variation or replacement easily figured out by a person skilled in the art within the technical scope disclosed in the present invention shall fall within the protection scope of the present invention. Therefore, the protection scope of the present invention shall be subject to the protection scope of the claims.

## Claims

1. A suture knot locking system, comprising a driving mechanism, a sheath, a tying head assembly disposed at a distal end of the sheath, a blade tube disposed inside the sheath, and a cutting portion disposed inside the sheath, wherein the tying head assembly comprises a suture threading channel communicated with the sheath; a cutting edge that is in contact fit with the cutting portion is disposed at a distal end of the blade tube; a suture extends into the sheath through the suture threading channel and is located between the cutting portion and the cutting edge; a proximal end of the blade tube is connected to the driving mechanism; in a process of operating the driving mechanism, the driving mechanism simultaneously drives the blade tube to move in an axial direction of the sheath relative to the sheath and drives the blade tube to rotate around an axis of the blade tube.

2. The suture knot locking system according to claim 1, wherein the driving mechanism comprises a screw-in member; the proximal end of the blade tube is connected to the screw-in member; and the screw-in member moves in the axial direction of the sheath and rotates around the axis of the blade tube.

3. The suture knot locking system according to claim 2, wherein the driving mechanism further comprises a fixing member; the fixing member is provided with a threaded hole extending in the axial direction of the sheath; the screw-in member is provided with an external thread and is threaded in the threaded hole; and the external thread is in threaded fit with the threaded hole.

4. The suture knot locking system according to claim 2, wherein the driving mechanism further comprises a moving member; the moving member moves in the axial direction of the sheath and is connected to a proximal end of the screw-in member;
the suture knot locking system further comprises a push rod; the push rod is disposed inside the blade tube; the screw-in member is provided with a through hole that extends through the proximal end and a distal end of the screw-in member; the proximal end of the blade tube is communicated with the through hole; a proximal end of the push rod extends out from the proximal end of the blade tube and is connected to the moving member through the through hole;
the push rod moves in the axial direction of the sheath under driving of the moving member; during movement of the push rod toward the distal end, a distal end of the push rod presses the tying head assembly; the tying head assembly has an initial state and a knot locking state; and the tying head assembly transitions from the initial state to the knot locking state under pressing action of the push rod, to lock the suture threaded in the tying head assembly.

5. The suture knot locking system according to claim 4, wherein the suture knot locking system further comprises a support sleeve; the support sleeve surrounds the outside of the push rod; a distal end of the support sleeve is communicated with the suture threading channel; and a proximal end of the support sleeve is fixed.

6. The suture knot locking system according to claim 4, wherein the tying head assembly comprises a tying head and a tying pin; the tying head has a pin hole extending through a proximal end and a distal end of the tying head; the suture threading channel comprises the pin hole;
in the initial state, the tying pin is located between the tying head and the push rod, and the tying pin moves inside the sheath under pushing of the push rod; and in the knot locking state, the tying pin is inserted into the pin hole.

7. The suture knot locking system according to claim 6, wherein the tying head assembly further comprises a tying head seat; the sheath is fixedly connected to the tying head seat; the tying head seat is provided with a seat hole extending through a proximal end and a distal end of the tying head seat; the tying head is detachably disposed in the seat hole; and the pin hole and the seat hole jointly define the suture threading channel.

8. The suture knot locking system according to claim 5, wherein a first suture passing hole is formed in an outer circumferential surface of the support sleeve; and the suture extends between the cutting portion and the cutting edge sequentially via the suture threading channel, the support sleeve, and the first suture passing hole.

9. The suture knot locking system according to claim 2, wherein the driving mechanism further comprises a knob; the knob surrounds the outside of the screw-in member; a sliding chute is formed in one of an inner circumferential surface of the knob and an outer circumferential surface of the screw-in member; a sliding block that is in sliding fit with the sliding chute is arranged on the other one of the inner circumferential surface of the knob and the outer circumferential surface of the screw-in member; and the sliding chute extends in the axial direction of the sheath.

10. The suture knot locking system according to any one of claims 1 to 9, wherein the cutting portion is disposed at a proximal end of the tying head assembly and is provided with a cutting surface facing the proximal end; the cutting edge is formed at the distal end of the blade tube and is opposite to the cutting surface;
alternatively, the cutting portion is disposed on an inner wall surface of the suture threading channel; and the cutting edge is formed on an outer circumferential surface of the blade tube.

11. The suture knot locking system according to any one of claims 1 to 9, wherein the blade tube comprises a main body section and a cutting section connected to a distal end of the main body section; the cutting edge is disposed at a distal end of the cutting section; and bending resistance of the cutting section is higher than bending resistance of the main body section.

12. The suture knot locking system according to any one of claims 1 to 9, wherein a second suture passing hole is formed in an outer circumferential surface of the sheath; and the suture is threaded out of the sheath sequentially via the suture threading channel, the sheath, and the second suture passing hole, so that the suture is at least partially disposed between the cutting portion and the cutting edge.
